(19) Europäisches Patentamt
European Patent Office
Office européen des brevets

(11) Veröffentlichungsnummer: **0 021 013**
**B1**

(12) # EUROPÄISCHE PATENTSCHRIFT

(45) Veröffentlichungstag der Patentschrift: **13.10.82**

(51) Int. Cl.³: **C 07 D 309/22**, C 11 B 9/00, A 61 K 7/46

(21) Anmeldenummer: **80102717.8**

(22) Anmeldetag: **16.05.80**

(54) **Neue 2,4-disubstituierte Pyranderivate, deren Herstellung und deren Verwendung als Riechstoffe.**

(30) Priorität: **21.06.79 DE 2925043**

(43) Veröffentlichungstag der Anmeldung: **07.01.81 (Patentblatt 81/01)**

(45) Bekanntmachung des Hinweises auf die Patenterteilung: **13.10.82 Patentblatt 82/41**

(84) Benannte Vertragsstaaten: **BE CH DE FR GB LI NL**

(73) Patentinhaber: **BASF Aktiengesellschaft**
**Carl-Bosch-Strasse 38**
**D-6700 Ludwigshafen (DE)**

(72) Erfinder: **Hoffmann, Werner, Dr. Chem.**
**Ringstrasse 11 c**
**D-6701 Neuhofen (DE)**
Erfinder: **Thoemel, Frank, Dr. Chem.**
**Leberstrasse 17**
**D-6940 Weinheim (DE)**

(56) Entgegenhaltungen:
**DE-C-1 221 388**
**US-A-4 071 535**
**Chemical Abstracts, Band 77, No. 7, 14. August 1972, Columbus, Ohio, USA A. AMANO et al. « 2-(2-Hydroxymethyl-1'-propenyl)-4-methyltetrahydropyran » page 469, column 1, Abstract No. 48248d**

**Chemical Abstracts, Band 77, No. 7, 14. August 1972, Columbus, Ohio, USA A. AMANO et al. « 2-(2-Formyl-1-propenyl)-4-methyltetrahydropyran » page 468, column 2, Abstract No. 48244z**

**Patents Abstracts of Japan, Band 1, Nr. 64 22 Juni 1977 Seite 889C77**

**EP 0 021 013 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 18, rue St-Denis, 75001 Paris, France

Neue 2,4-disubstituierte Pyranderivate, deren Herstellung und deren Verwendung als Riechstoffe

Die Erfindung betrifft neue 2,4-disubstituierte Pyranderivate der allgemeinen Formel I

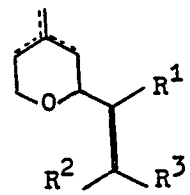

(I)

in der

$R^1$ und $R^2$ jeweils für Wasserstoff oder eine Methylgruppe stehen ; und

$R^3$ eine der Gruppen $-CH_2OH$, $-CH_2$-O-$COCH_3$, $-CH_2-OCHO$, $-CH_2-O-COC_2H_5$, $-COOCH_3$, $-COOC_2H_5$, $-COOC_3H_7$, $-CH_2Cl$, $CH_2Br$, $-CH_2J$ oder CHO bedeutet und die im Bereich der gestrichelten Linie eine Doppelbindung aufweisen sowie deren Verwendung als Riechstoffe.

Unter den in der Natur vorkommenden Verbindungen vom Pyrantyp hat das sogenannte Rosenoxid (Formel IV) wegen seiner Dufteigenschaften eine besondere Bedeutung erlangt.

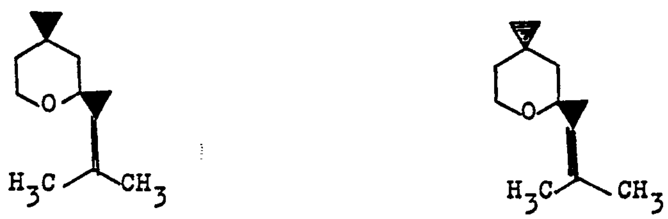

(IV)

cis trans

Es hat daher nicht an Versuchen gefehlt, diese Komponente des bulgarischen Rosenöls (enthält ca. 1 % (−)-cis-Verbindung) oder des Öls von Geranium bourbon (enthält ca. 1 % (−)-cis- und (−)-trans-Verbindung) zu synthetisieren (siehe beispielsweise Zeitschrift für Chemie *11* (1971), 459).

Von diesem natürlichen Vorbild leiten sich einige rein synthetische Verbindungen mit Rosenduft oder ähnlichen Dufteigenschaften ab.

So ist es z.B. aus der DE-PS 12 21 388 bekannt durch Kondensation von 2-Methyl-2,4-pentandiol mit Mesityloxid in Gegenwart von Schwefelsäure ein Pyran der Formel V

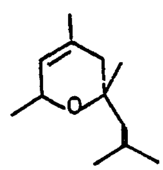

(V)

herzustellen, welches eine Rosen- und Geraniumnote aufweist.

Die Synthese dieses Pyrans erfolgte nach einem Verfahren, welches von Williams et al. (s. J. Am. Chem. Soc. *72* (1950), S. 5738) für die Kondensation von Aldehyden oder Ketonen in Gegenwart saurer Katalysatoren mit 2-Methyl-2,4-pentandiol oder 4-Methyl-4-penten-2-ol zu den entsprechenden Pyranderivaten beschrieben wurde. Weitere Erkenntnisse hinsichtlich der entstehenden Produkte und des Mechanismus dieser Cyclokondensationsreaktion wurden in Tetrahedron 1957, Vol. 1, S. 284-88 dargelegt.

Ferner ist es aus den US-Patentschriften 4 070 491 und 4 071 535 bekannt, bestimmte 2-Alkyl-4-phenyl-dihydropyrane zur Verbesserung der organoleptischen Eigenschaften von Nahrungsmitteln und Tabak zu verwenden.

Aus den japanischen Patentveröffentlichungen
72/14382 (C.A. *77*, S. 468 Ref. 48244 z) und
72/14383 (C.A. *77*, S. 469 Ref. 48248 d)
waren die Verbindungen VIA und VIb

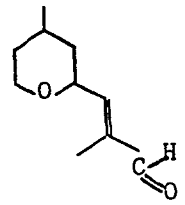

VIa

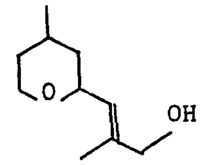

VIb

bekannt, die einen blumigen bzw. pilzartigen Geruch aufweisen. Demgegenüber stellen die erfindungsgemäßen neuen Verbindungen eine Bereicherung des Gebiets der Duftstoffe dar, zumal man aus der Kenntnis bekannter Verbindungen keine Vorhersage über die Duftstoffeigenschaften neuer Verbindungen machen kann.

Es wurde nun überraschenderweise gefunden, daß sich auch α,β-ungesättigte Aldehyde, die außer der Formylgruppe noch weitere funktionelle Gruppen wie Estergruppen, eine Hydroxymethylgruppe, eine Formylgruppe oder eine Halogenmethylgruppe in β-Stellung enthalten, sich ohne besondere Schwierigkeiten und in guten Ausbeuten mit 3-Methyl-3-buten-1-ol in Gegenwart saurer Katalysatoren zu den entsprechenden 2,4-disubstituierten Pyranderivaten cyclisieren lassen und dabei neue Verbindungen mit interessanten Duftnoten gebildet werden.

Gegenstand der Erfindung ist daher auch ein Verfahren zur Herstellung der neuen Pyranderivate der allgemeinen Formel I, das dadurch gekennzeichnet ist, daß man 3-Methyl-3-buten-1-ol (II)

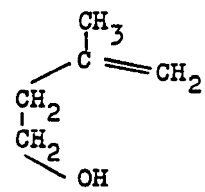

(II)

in Gegenwart saurer Katalysatoren mit Aldehyden der allgemeinen Formel III

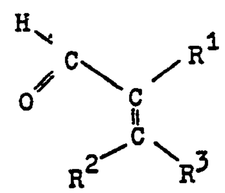

(III)

in der

$R^1$, $R^2$ und $R^3$ die oben angegebene Bedeutung haben, umsetzt.

Zur Herstellung der Verbindung der Formel I, in der $R^3$ für $—CH_2OH$ steht, wird am besten das entsprechende Acetat in üblicher Weise verseift oder vorteilhafter einer Umesterung, beispielsweise mit Methanol in Gegenwart von Natriummethylat, unterworfen.

Als α,β-ungesättigte Aldehyde der allgemeinen Formel V, die weitere funktionelle Gruppen enthalten, kommen z.B. in Betracht: 2-Methyl- sowie 3-Methyl-4-acetoxy-2-buten-1-al ; 3-Formylcrotonsäure-methylester und -äthylester, 2-Methyl-4-chlor-2-buten-1-al, 2-Methyl-4-brom-2-buten-1-al und 4-Acetoxy-2-buten-1-al.

Das als weitere Reaktionskomponente erforderliche 3-Methyl-but-3-en-1-ol (IV) ist eine handelsübliche Verbindung, die durch Prins-Reaktion von Isobutylen und Formaldehyd hergestellt werden kann.

Als saure Katalysatoren kommen starke anorganische Säuren, wie $H_2SO_4$ oder HCl ; starke organische Säuren, wie p-Toluol-sulfonsäure und Oxalsäure, stark saure Ionenaustauscher sowie Brönnstedtsäuren, wie wasserfreies $FeCl_3$ oder $ZnCl_2$ in Betracht. Mit besonderem Vorteil arbeitet man mit p-Toluolsulfonsäure. Den sauren Katalysator verwendet man im allgemeinen in Mengen von 0,1 mmol bis 100 mmol, vorzugsweise 1 bis 10 mmol, bezogen auf 1 mol 3-Methyl-3-buten-1-ol.

Die Umsetzung wird im allgemeinen in einem Lösungsmittel durchgeführt, man kann jedoch auch lösungsmittelfrei arbeiten, wobei jedoch mehr Polymere als Nebenprodukte gebildet werden.

Als Lösungsmittel kommen beispielsweise in Betracht: Methylenchlorid, Cyclohexan, Toluol und Hexan.

Die Umsetzung erfolgt im allgemeinen bei Temperaturen von 50 bis 150 °C, vorzugsweise 80 bis

120 °C. Die Reaktionszeit beträgt je nach Reaktant und Reaktionstemperatur 0,5 bis 24, vorzugsweise 1 bis 4 Stunden.

Die erfindungsgemäßen 2,4-disubstituierten Pyranderivate besitzen sehr interessante organoleptische Eigenschaften. Sie sind Duftstoffe mit sehr interessanten grünen Duftnoten, die einen würzigen, krautigen oder aromatischen Einschlag aufweisen. Die neuen Dufstoffe eignen sich für kosmetische Zubereitungen aller Art sowie für Wasch- und Reinigungsmittel bzw. sind wertvolle Duftverbesserer für technische Produkte. Sie können entweder in reiner Form oder in Mischung mit anderen Duftstoffen verwendet werden.

Mit Hilfe des erfindungsgemäßen Verfahrens sind die neuen Verbindungen auf einfache und wirtschaftliche Weise erhältlich.

## Beispiel 1

43 g (0,5 mol) 3-Methyl-3-buten-1-ol und 71 g (0,5 mol) 2-Methyl-4-acetoxy-2-buten-1-al wurden in einer Mischung aus 200 ml Toluol und 200 ml Hexan gelöst, mit 0,5 g p-Toluolsulfonsäure versetzt und 2 Stunden unter Rückfluß zum Sieden erhitzt, wobei 10,5 g Wasser azeotrop abdestilliert wurden. Das Reaktionsgemisch wurde danach mit Wasser neutral gewaschen und die Lösungsmittel bei 30 °C und 10 mbar abdestilliert. Der Rückstand wurde fraktioniert destilliert. Dabei erhielt man 81 g Hauptlauf (77 % d. Th.), der laut NMR-Spektrum ca. 80 Gew.-% 2-(1-Methyl-3-acetoxy-1-propen-1-yl)-4-methylen-tetrahydropyran und je ca. 10 Gew.-% 2-(1-Methyl-3-acetoxy-1-propen-1-yl)-4-methyl-5,6-dihydro-2H-pyran und 2-(1-Methyl-3-acetoxy-1-propen-1-yl)-4-methyl-2,3-dihydro-6H-pyran enthielt.

Der Siedepunkt des erhaltenen Gemisches betrug 75-78 °C bei 0,01 mbar;

$n_D^{25}$ = 1,489 0;

Duftnote: grün, dillartig, Gurkenkraut.

## Beispiel 2

515 g (2,45 mol) eines gemäß Beispiel 1 erhaltenen Pyrangemisches wurden in 416 g (13 mol) Methanol gelöst, die Lösung mit 3 g einer 30 %igen Lösung von Natriummethylat in Methanol als Katalysator versetzt und unter Rückfluß zum Sieden erhitzt. Der bei dieser Umesterungsreaktion entstehende Essigsäuremethylester wurde über eine 50 cm Glasfüllkörperkolonne kontinuierlich abdestilliert. Nach Beendigung der Essigesterbildung wurde das überschüssige Methanol abdestilliert und das Reaktionsprodukt fraktioniert destilliert. Man erhält 392 g Hauptlauf, der laut NMR-Spektrum in der Zusammensetzung der Isomeren dem Ausgangsmaterial entspricht (Ausbeute 94 % der Theorie);

Sdp. = 87-90 °C bei 0,04 mbar;

$n_D^{25}$ = 1,501 1;

Duftnote: krautig, würzig.

## Beispiel 3

43 g (0,5 mol) 3-Methyl-3-buten-1-ol und 71 g (0,5 mol) 3-Formyl-crotonsäureäthylester wurden in 200 ml Toluol gelöst, die Lösung mit 0,3 g p-Toluolsulfonsäure versetzt und 1,5 Stunden unter Rückfluß zum Sieden erhitzt, wobei 9 ml Wasser azeotrop abdestilliert wurden. Anschließend wurde das Reaktionsgemisch mit Wasser neutral gewaschen, das Lösungsmittel bei 30 °C und 10 mbar entfernt und der Rückstand fraktioniert destilliert. Man erhielt 70 g Hauptlauf (63 % d. Th.), der laut NMR-Spektrum ca. 70 % 2-(1-Methyl-2-carbäthoxy-1-äthen-1-yl)-4-methylen-tetrahydropyran, ca. 20 % 2-(1-Methyl-2-carbäthoxy-1-äthen-1-yl)-4-methyl-5,6-dihydro-2H-pyran und ca. 10 % 2-(1-Methyl-2-carbäthoxy-1-äthen-1-yl)-4-methyl-2,3-dihydro-6H-pyran enthält;

Sdp. 92-95 °C bei 0,15 mbar;

$n_D^{25}$ = 1,487 8;

Duftnote: grün, fruchtig.

## Beispiel 4

86 g (1 mol) 3-Methyl-3-buten-1-ol und 118,5 g (1 mol) 2-Methyl-4-chlor-2-buten-1-al wurden in 300 ml Toluol gelöst, die Lösung mit 0,5 g p-Toluolsulfonsäure versetzt und 3 Stunden unter Rückfluß zum Sieden erhitzt, wobei 18 ml Wasser azeotrop abdestillierten.

Das Reaktionsgemisch wurde anschließend mit Natriumbicarbonatlösung neutral gewaschen, das Toluol bei 30 °C und 10 mbar abdestilliert und der Rückstand fraktioniert destilliert. Man erhielt 131 g Hauptlauf (70 % d. Th.), der laut NMR-Spektrum ca. 60 % 2-(1-Methyl-3-chlor-1-propen-1-yl)-4-methylen-tetrahydropyran, ca. 30 % 2-(1-Methyl-3-chlor-1-propen-1-yl)-4-methyl-5,6-dihydro-2H-pyran und ca. 10 % 2-(1-Methyl-3-chlor-1-propen-1-yl)-4-methyl-2,3-dihydro-6H-pyran enthielt;

Sdp. 56-64 °C bei 1 mbar;

$n_D^{25}$ = 1,501 0;

Duftnote: würzig, aromatisch, grün.

Beispiel 5

86 g (1 mol) 3-Methyl-3-buten-1-ol und 143 g (0,78 mol) 77 proz. 3-Methyl-4-acetoxy-2-buten-1-al wurden in 1,4 l Toluol gelöst, die Lösung mit 1,4 g p-Toluolsulfonsäure versetzt und 1 Stunde unter Rückfluß zum Sieden erhitzt, wobei 19 ml Wasser azeotrop abdestillierten. Das Reaktionsgemisch wurde anschließend mit verdünnter Natronlauge neutral gewaschen, das Toluol bei 30 °C und 10 mbar abdestilliert und der Rückstand fraktioniert destilliert. Man erhielt 103 g Hauptlauf (63 % d. Th.). Die Isomeren lassen sich laut NMR-Spektrum und GC-Analyse folgendermaßen zuordnen : 56 % 2-(2-Methyl-3-acetoxy-1-propen-1-yl)-4-methylen-tetrahydropyran, 21 % 2-(2-Methyl-3-acetoxy-1-propen-1-yl)-4-methyl-5,6-dihydro-2H-pyran und 23 % 2-(2-Methyl-3-acetoxy-1-propen-1-yl)-4-methyl-2,3-dihydro-6H-pyran ;
Sdp. 75-81 °C bei 0,1 mbar ;
$n_D^{25}$ = 1,477 6 ;
Duftnote : citrusartig, leicht blumig.

Beispiel 6

86 g (1 mol) 3-Methyl-3-buten-1-ol und 128 g (1 mol) 4-Acetoxy-3-buten-1-al wurden in 250 ml Toluol gelöst, die Lösung mit 0,5 g p-Toluolsulfonsäure versetzt und 3 Stunden unter Rückfluß zum Sieden erhitzt, wobei 19 ml Wasser azeotrop abdestillierten. Das Reaktionsgemisch wurde anschließend mit verdünnter Natronlauge neutral gewaschen, das Toluol bei 30 °C und 10 mbar abdestilliert und der Rückstand fraktioniert destilliert. Man erhielt 112,5 g Hauptlauf (62 % d. Th.). Die Isomeren lassen sich laut NMR-Spektrum in Verbindung mit der GC-Analyse folgendermaßen zuordnen : ca. 65 % 2-(3-Acetoxy-1-propen-1-yl)-4-methylen-tetrahydropyran, ca. 30 % 2-(3-Acetoxy-1-propen-1-yl)-4-methyl-5,6-dihydro-2H-pyran und ca. 5 % 2-(3-Acetoxy-1-propen-1-yl)-4-methyl-2,3-dihydro-6H-pyran ;
Sdp. 78-80 °C bei 0,1 mbar ;
$n_D^{25}$ = 1,477 8 ;
Duftnote : blumig, süßlich, frisch, Richtung Bergamott.

**Ansprüche**

1. 2,4-disubstituierte Pyranderivate der allgemeinen Formel I

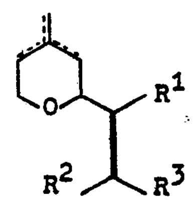

(I)

in der
$R^1$ und $R^2$ jeweils für Wasserstoff oder eine Methylgruppe stehen ; und
$R^3$ eine der Gruppen $-CH_2OH$, $-CH_2-O-COCH_3$, $-CH_2-OCHO$, $-CH_2-O-COC_2H_5$, $-COOCH_3$, $-COOC_2H_5$, $-COOC_3H_7$, $-CH_2Cl$, $-CH_2-Br$, $-CH_2J$ oder $-CHO$ bedeutet und die im Bereich der gestrichelten Linie eine Doppelbindung aufweisen.

2. Verfahren zur Herstellung der neuen Pyranderivate der allgemeinen Formel I gemäß Anspruch 1, dadurch gekennzeichnet, daß man 3-Methyl-3-buten-1-ol (II)

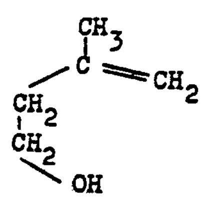

in Gegenwart saurer Katalysatoren mit Aldehyden der allgemeinen Formel III

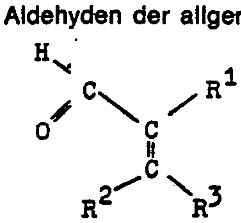

(III)

in der

$R^1$, $R^2$ und $R^3$ die oben angegebene Bedeutung haben, umsetzt.

3. Verwendung der neuen Pyranderivate der allgemeinen Formel I gemäß Anspruch 1 als Riechstoff.

## Claims

1. 2,4-Disubstituted pyran derivatives of the general formula I

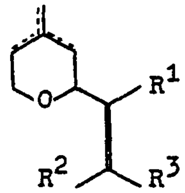

(I)

where

$R^1$ and $R^2$ are each hydrogen or methyl and

$R^3$ is $-CH_2OH$, $-CH_2-O-COCH_3$, $-CH_2OCHO$, $-CH_2-O-COC_2H_5$, $-COOCH_3$, $-COOC_2H_5$, $-COOC_3H_7$, $-CH_2Cl$, $-CH_2Br$, $-CH_2I$ or $-CHO$, and which possess a double bond within the region shown by the broken line.

2. A process for the preparation of a novel pyran derivative of the general formula I as claimed in claim 1, characterized in that 3-methyl-but-3-en-1-ol (II)

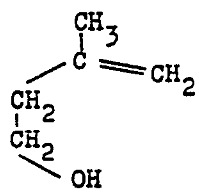

is reacted with an aldehyde of the general formula III

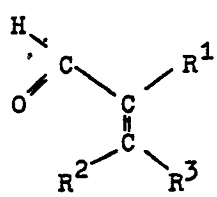

(III)

where

$R^1$, $R^2$ and $R^3$ have the above meanings in the presence of an acid catalyst.

3. Use of a novel pyran derivative of the general formula I as claimed in claim 1 as a fragrance.

## Revendications

1. Dérivés du pyranne 2,4-disubstitués de la formule générale I

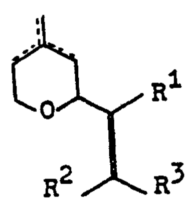

(I)

dans laquelle

$R^1$ et $R^2$ désignent chacun un atome d'hydrogène ou un radical méthyle et

$R^3$ représente l'un des groupes $-CH_2OH$, $-CH_2-O-COCH_3$, $-CH_2-OCHO$, $-CH_2-O-COC_2H_5$, $-COOCH_3$, $-COOC_2H_5$, $-COOC_3H_7$, $-CH_2Cl$, $-CH_2Br$, $-CH_2I$ ou $-CHO$, comportant une double liaison dans la région indiquée par les lignes en trait interrompu.

2. Procédé de préparation des nouveaux dérivés du pyranne de la formule générale I suivant la revendication 1, caractérisé en ce que l'on fait réagir le méthyl-3 butène-3 ol-1 (II)

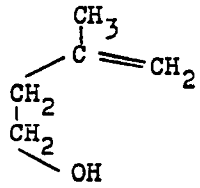

en présence d'un catalyseur acide avec un aldéhyde de la formule générale III

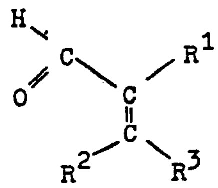

(III)

dans laquelle

$R^1$, $R^2$ et $R^3$ possèdent les significations définies.

3. Utilisation des nouveaux dérivés du pyranne de la formule générale I selon la revendication 1 comme agents parfumants.